# EUROPEAN PATENT APPLICATION

(11) **EP 4 238 612 A1**
(43) Date of publication of application: **06.09.2023**
(21) Application number: 21886089.8
(22) Date of filing: 22.10.2021
(51) Int. Cl.: A61Q 1/00, C09D 11/17, A61K 8/81

(54) **MARKER COMPOSITION FOR SKIN**

(30) Priority: 27.10.2020 JP 2020180035
(71) Applicant: MITSUBISHI PENCIL COMPANY, LIMITED, Tokyo 140-8537 (JP)
(72) Inventor: INOUE Kensuke, Fujioka-shi, Gunma 375-8501 (JP); SAGAWA Wataru, Tokyo 140-8537 (JP); HAGA Hisato, Tokyo 140-8537 (JP)
(74) Representative: Vossius & Partner Patentanwälte Rechtsanwälte mbB
(86) International application number: PCT/JP2021/039104
(87) International publication number: WO 2022/091971

(57) **Abstract**

A marker composition for skin using a dye with good color development, which is capable of preventing color separation and also preventing absorption of the dye into the skin or the like, is provided. The marker composition for skin contains at least 1 to 5 mass% of an acid dye, an aminoalkyl (meth)acrylate copolymer, a water-soluble organic solvent, and water, wherein a pH is lower than 7.0, the aminoalkyl (meth)acrylate copolymer being a copolymer polymerized from monomers selected from Group A and from Group B respectively, and a ratio of the monomer of the group B being 2 to 20 mass% in the copolymer;
Group A: ethyl (meth)acrylate, methyl (meth)acrylate, n-propyl (meth)acrylate, and the like;
Group B: 2-(dimethylamino)ethyl (meth)acrylate, 2-(diethylamino)ethyl (meth)acrylate, and 3-(dimethylamino)propyl (meth)acrylate.

## Description

### Technical Field

The present specification relates to a marker composition for skin, the composition capable of preventing color separation using a dye with good color development and also preventing absorption of the dye into the skin or the like.

### Background Art

Examples of marker compositions for skin known in the art include:
(1) an ink composition for skin marking for directly marking the skin of a human body, the ink composition including: at least one component of ethyl alcohol, isopropyl alcohol, normal propyl alcohol, water, or the like as a solvent; at least one component of glycerin, propylene glycol, poly(oxyethylene) or a its derivative, or poly(oxypropylene) or its derivative as a modifier; and a low-toxic food dye or the like (see, e.g., Patent Document 1);
(2) an ink composition for skin marking for directly marking the skin of a human body, the ink composition including: at least one or two or more components of ethyl alcohol, isopropyl alcohol, normal propyl alcohol, water, or the like as a solvent(s); at least one or two or more components of glycerin, propylene glycol, poly(oxyethylene) or its derivative, or poly(oxypropylene) or its derivative as a modifier(s); and one or two or more dyes of basic dyes (see, e.g., Patent Document 2);
(3) a makeup cosmetic characterized by blending an emulsifier-free polymer resin emulsion (see, e.g., Patent Document 3);
(4) a makeup cosmetic characterized by blending a copolymer emulsion obtained from one or two or more acrylic ester monomers having 4 to 18 carbons in the ester moiety and one or two or more methacrylic ester monomers (see, e.g., Patent Document 4); and
(5) an aqueous coating composition to be used as a face paint, a body paint, or the like, the aqueous coating composition containing: an aqueous isoprene rubber latex having an isoprene rubber component; and a colorant (see, e.g., Patent Document 5).

The ink compositions for skin marking of Patent Documents 1 and 2 described above include a "low-toxic food dye (Japanese Pharmacopoeia dye) " or a "basic dye", which can be used for marking during surgical operations, marking on foods, and the like. These inks firmly adhere to the skin or the like during use and can be easily removed, and are harmless even if they are absorbed by a body through the skin or the like and enter the body. However, although these ink compositions are non-toxic even if they enter the body by absorption through the skin or the like, the designs of these ink compositions do not encompass prevention of the ink from entering the body. Therefore, further improvement has been desired.
In addition, Patent Documents 3 and 4 describe compositions that can be used for body paints. Their approaches include the use of a pigment to prevent absorption into the skin. In these documents, the compositions use an emulsion resin for film formation, especially an acrylic emulsion resin, which can provide good film formation properties. These films have good properties, such as rubbing resistance and good water resistance, and thus has excellent "makeup durability". However, these compositions are obtained using mainly an iron oxide pigment which has a specific gravity greatly different from the emulsion, and this difference is likely to cause what is called color separation and problems, such as poor stability.
Patent Document 5 above describes use of a film-forming synthetic isoprene rubber latex to eliminate uncomfortable feeling, such as "tight feeling" of the skin, and also exemplify dyes as colorants. Patent Document 5 above describes that the use of a synthetic isoprene rubber latex allows excellent elasticity and flexibility: however, the rubber film shrinks, and the shrinkage causes "feeling of being pulled" and is also presumed to have poor weather resistance against direct sunlight and the like.

### Citation List

### Patent Document

Patent Document 1: JP S55-137175 A (Claims, Description, etc.)
Patent Document 2: JP S58-154772 A (Claims, Description, etc.)
Patent Document 3: JP S54-049338 A (Claims, Description, etc.)
Patent Document 4: JP S54-151139 A (Claims, Description, etc.)
Patent Document 5: JP 2019-002002 A (Claims, Description, etc.)

### Summary of Invention

### Technical Problem

The present disclosure has been made in view of these issues, and current status of the art described above, to solve them. An object of the present disclosure is to provide a marker composition for skin, the composition capable of preventing color separation using a dye with good color development and also preventing absorption of the dye into the skin or the like.

### Solution to Problem

As a result of diligent studies in view of the above issues of the art, the present inventors have found that the intended marker composition for skin is obtained by making it include at least a predetermined amount of an acid dye, a specific copolymer, a water-soluble organic solvent, and water, and making its pH lower than a specific value. And thus the present disclosure is completed.

That is, a marker composition for skin of the present disclosure contains at least 1 to 5 mass% of an acid dye, and an aminoalkyl (meth)acrylate copolymer, a water-soluble organic solvent, and water, wherein a pH is lower than 7.0, the aminoalkyl (meth) acrylate copolymer is a copolymer polymerized from each monomer selected from the group A and from the group B respectively, a ratio of the monomer of the group B being 2 to 20 mass% in the copolymer:
Group A:
   ethyl (meth)acrylate, methyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate;
Group B:
   2-(dimethylamino)ethyl (meth)acrylate, 2-(diethylamino)ethyl (meth)acrylate, and 3-(dimethylamino)propyl (meth)acrylate. The acid dye is preferably a legally-approved cosmetic dye. Here, the "legally-approved cosmetic dye" refers to an organic synthetic dye that can be used in pharmaceutical products, quasi-pharmaceutical products, and cosmetics as defined by the Ministry of Health, Labour and Welfare in 1996 and 83 types in total are classified into Group I (11 types), Group II (47 types), or Group III (25 types). The water-soluble organic solvent is preferably a lower alcohol having 3 or less carbons.

### Advantageous Effects of Invention

According to the present disclosure, there is provided the marker composition for skin, the composition capable of preventing color separation using a dye with good color development and also preventing absorption of the dye into the skin or the like.
The object and effects of the present disclosure can be recognized and obtained particularly using the components and combinations indicated in the claims. Both general explanation described above and detailed explanation described below are exemplary and explanatory and do not limit the present disclosure described in Claims. Description of Embodiments

Embodiments of the present disclosure will be described below in detail. However, note that the technical scope of the present disclosure is not limited to the embodiments described below and includes the invention described in Claims and equivalents thereof.

A marker composition for skin of the present disclosure contains at least 1 to 5 mass% of an acid dye, an aminoalkyl (meth)acrylate copolymer, a water-soluble organic solvent, and water, in which a pH is lower than 7.0, the aminoalkyl (meth)acrylate copolymer is a copolymer polymerized from monomers selected from the group A and from the group B below respectively, and a ratio of the monomer of the group B is 2 to 20 mass% in the copolymer:
Group A:
   ethyl (meth) acrylate, methyl (meth) acrylate, n-propyl (meth) acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group B:
   2-(dimethylamino)ethyl (meth)acrylate, 2-(diethylamino)ethyl (meth)acrylate, and 3-(dimethylamino)propyl (meth)acrylate.

Examples of the acid dye used in the present disclosure include general acid dyes, and preferably, at least one of acid dyes among legally-approved cosmetic dyes can be used. Specific examples of the acid dye include at least one (each alone or a mixture of two or more, the same hereinafter) of Red No. 2 (C. I. 16185), Red No. 3 (C. I. 45430), Red No. 102 (C. I. 16255), Red No. 104 (C. I. 45410), Red No. 105 (C. I. 45440), Red No. 106 (C. I. 45100), Yellow No. 4 (C. I. 19140), Yellow No. 5 (C. I. 15985), Green No. 3 (C. I. 42053), Blue No. 1 (C. I. 42090), Blue No. 2 (C. I. 73015), Red No. 201 (C. I. 15850), Red No. 206 (C. I. 15630: 1), Red No. 227 (C. I. 17200), Red No. 230 (C. I. 45380), Red No. 231 (C. I. 45410), Red No. 232 (C. I. 45440), Orange No. 205 (C. I. 15510), Orange No. 207 (C. I. 45425), Yellow No. 202 (C. I. 45350), Yellow No. 203 (C. I. 47005), Green No. 201 (C. I. 61570), Green No. 204 (C. I. 59040), Green No. 205 (C. I. 42095), Blue No. 202 (C. I. 42052), Blue No. 203 (C. I. 42052), Blue No. 205 (C. I. 42090), Brown No. 2011 (C. I. 20170), Red No. 401 (C. I. 45190), Red No. 502 (C. I. 16155), Red No. 503 (C. I. 16150), Red No. 504 (C. I. 14700), Red No. 506 (C. I. 15620), Orange No. 403 (C. I. 12100), Orange No. 402 (C. I. 14600), Yellow No. 402 (C. I. 18950), Yellow No. 403 (1) (C. I. 10316), Yellow No. 406 (C. I. 13065), Yellow No. 407 (C. I. 18820), Green No. 401 (C. I. 10020), Green No. 402 (C. I. 42085), Purple No. 401 (C. I. 60730), or Black No. 401 (C. I. 20470). Among these acid dyes, combining, for example, a red dye, a yellow dye, a blue dye, or the like enables color preparation from yellow, brown to black and any color designing.

The (total) content of these acid dyes is desirably 1 to 5 mass% (hereinafter, "mass%" is referred to simply as "%") and preferably 1 to 3% relative to the total amount of the marker composition for skin.
When the content of the acid dye is less than 1%, sufficient color-developing property is not provided, while when the marker composition for skin with content is more than 5%, sufficient water resistance is not provided and the absorption of the dye into the skin or the like is affected; which is not preferred.

The aminoalkyl (meth) acrylate copolymer used in the present disclosure is a component to prevent color separation despite the use of an acid dye with good color development and also to prevent absorption of the dye into the skin or the like. The examples include at least one of a 2-(dimethylamino)ethyl methacrylate-2-ethylhexyl acrylate-ethyl methacrylate copolymer and a 2-(dimethylamino)ethyl methacrylate-methyl methacrylate-n-butyl methacrylate copolymer.

The aminoalkyl (meth)acrylate copolymer of the present disclosure is a copolymer polymerized from at least one each monomer selected from the group A and from the group B respectively. From the viewpoints of water resistance and absorption of the dye into the skin or the like, the ratio of the amine monomer of the group B is indispensably 2 to 20%, preferably 8 to 15%, in the copolymer (monomer of group A + monomer of group B + polymerization initiator and the like described later) . If the content of the amine monomer is more than 20%, the marker composition would be easily shed from skin or the like with water. On the other hand, if the content of the amine monomer is less than 2%, the absorption of the dye into the skin or the like would be affected, which is not preferred.
Group A:
   ethyl (meth) acrylate, methyl (meth) acrylate, n-propyl (meth) acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate
Group B:
   2-(dimethylamino)ethyl (meth)acrylate, 2-(diethylamino)ethyl (meth)acrylate, and 3-(dimethylamino)propyl (meth)acrylate

The wording " (meth) acrylate" represents "acrylate and/or methacrylate" in the group A above and the like. Particularly preferably, ethyl (meth) acrylate, which further improves adhesion of drawn lines to the skin or the like, is desirably used.
In the group B above, particularly preferably, 2- (dimethylamino) ethyl methacrylate is desirably used.

For the aminoalkyl (meth)acrylate copolymer used in the present disclosure, a commercially available product can be used if available, and preferably, an aminoalkyl (meth)acrylate copolymer obtained by the following production method is desirably used.
The aminoalkyl (meth) acrylate copolymer used in the present disclosure can be produced by procedures of dissolving mixed monomers containing at least one monomer selected from the group A and at least one monomer selected from the group B in a solvent, such as n-propanol; and performing solution polymerization using azobisisobutyronitrile or the like as a polymerization initiator or further using a reducing agent in the polymerization initiator in combination, further using a polymerizable surfactant if necessary.

In the present disclosure, for the content of the monomer of the group B among the components of the aminoalkyl (meth)acrylate copolymer, the above preferred ratio of the content is desirable from the viewpoints of color-developing properties, weather resistance, and stability, and the above content (2 to 20%) is set from the viewpoints, such as water resistance and absorption to the skin or the like.

In the present disclosure, the aminoalkyl (meth)acrylate copolymer used in the present disclosure is obtained in a content of 10 to 35% as a resin solid as the preferred aspect, specifically, by dissolving the mixed monomers containing at least the monomer of the group A and the monomer of the group B in n-propanol or the like and performing solution polymerization.
The aminoalkyl (meth)acrylate copolymer obtained is useful for adhesion and water resistance.

Examples of the water-soluble organic solvent used in the present disclosure include lower alcohols having 5 or less carbons. In particular, such a lower alcohol can reduce the solubility of the acid dye and the aminoalkyl (meth) acrylate copolymer to be obtained, and the viscosity of the composition containing these. This is preferred for allowing the composition to flow out when the composition is filled into an applicator and used. In addition, such a lower alcohol is also preferred for the drying property of the film after application. Specific examples of the water-soluble organic solvent include at least one (each alone or a mixture of two or more) of methyl alcohol (methanol), ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, and propylene glycol. Preferably, a lower alcohol having 3 or less carbons, particularly ethyl alcohol (ethanol), n-propyl alcohol, or isopropyl alcohol, is desirably used from the viewpoints of safety and ease of handling.

Desirably, the content of the water-soluble organic solvent used is preferably 40 to 80%, more preferably 50 to 70% relative to the total amount of the marker composition for skin from the viewpoints of the solubility of the aminoalkyl (meth)acrylate copolymer to be obtained and the solubility of the acid dye.
The content of this water-soluble organic solvent is 40% or greater, and it can improve the solubility of the aminoalkyl (meth)acrylate copolymer obtained and provide a stable solution, and in addition exhibits an antiseptic effect although the effect is slight. On the other hand, the content of this water-soluble organic solvent is 80% or less, and this further improves the solubility of the acid dye and can exhibit effects in terms of color-developing properties and water resistance.

For water serving as a solvent used in the present disclosure, distilled water, ion-exchanged water, purified water, pure water, ultrapure water, or the like can be used. From the viewpoints of the solubility of the aminoalkyl (meth) acrylate copolymer and the solubility of the acid dye, desirably, the amount of water is preferably 10 to 35%, more preferably 15 to 30% relative to the total amount of the marker composition for skin.

The marker composition for skin of the present disclosure contains at least an acid dye, aminoalkyl (meth)acrylate copolymer, a water-soluble organic solvent, and water. Moreover, a pH adjuster, a surfactant, a viscosity modifier, or the like can be appropriately used from the viewpoints of further improvement in dispersibility and stability of each dissolved component if necessary.

The marker composition for skin of the present disclosure preferably has a viscosity of 10 to 30 mPa·s at a temperature of 25°C as measured with a cone-plate viscometer standard cone rotor, a cone angle of 1°34', a cone radius of 24 mm, a shear rate of 76.6 s⁻¹, TVE-25L available from Toki Sangyo Co., Ltd. from the viewpoints of stability and applicability.
A more preferred range in the above viscosity range varies according to the types of blended components of the marker composition for skin but is desirably more preferably 15 to 25 mPa·s.
With a viscosity value of 10 mPa·s or higher, the marker composition can be applied to the skin or the like without causing liquid dripping, while with a viscosity value of 30 mPa·s or lower, the marker composition can be smoothly applied to the skin.
In addition, the viscosity can be adjusted to the above range by suitably combining the amount of each component, such as the acid dye, the aminoalkyl (meth) acrylate copolymer, water, and if necessary the viscosity modifier.

The pH of the marker composition for skin of the present disclosure is adjusted to lower than 7.0 to improve coloring properties, prevent skin irritation, and stabilize dissolution, and desirably, the pH is preferably adjusted to 4 or higher and lower than 7.
The marker composition for skin with a pH of 7 or higher may reduce dyeing affinity to the skin, and the marker composition for skin with a pH lower than 4 may cause skin irritation.
In the present disclosure, the pH can be adjusted by using a pH adjuster, including an organic acid, such as malic acid, citric acid, or glycolic acid; an inorganic acid, such as 5 M hydrochloric acid; a salt thereof; or aminomethyl propanol.

In the present disclosure, when a pH adjusting agent is used, more preferably, the blending ratio of the aminoalkyl (meth)acrylate copolymer to the pH adjuster in terms of mass ratio is desirably 15:5 to 15:1 and particularly preferably, desirably 15:3 to 15:1.
With the ratio of 15:3 to 15:1, the marker composition for skin has low irritation to the skin in a weakly acidic region where the pH is lower than 7.0.

The marker composition for skin of the present disclosure can be prepared by a usual method and can be produced by blending each component, such as at least one of the acid dyes, the aminoalkyl (meth)acrylate copolymer, water, and the water-soluble organic solvent, in the range of each content described above and uniformly stirring and mixing them.
For example, the marker composition for skin can be prepared by stirring the acid dye and a solvent, such as water, with a commonly used disperser or the like until the mixture is uniform, then mixing the aminoalkyl (meth)acrylate copolymer, then adding a pH adjuster, a viscosity modifier, or the like if necessary, and stirring with a homomixer or the like until the mixture is uniform with a disper or the like.

When the marker composition for skin of the present disclosure thus constituted is used, an applicator that facilitates the making of a drawing or the like on a skin site by applying to a skin portion (skin), such as a face surface or a body surface, to dye the skin can be used without particular limitation. For example, an applicator for skin, such as a container with a brush, a container with a spatula, a bottle, or a tube, can be used. The shape, structure, and the like of the applicator for skin to be used are not particularly limited.

The marker composition for skin of the present disclosure thus constituted contains at least 1 to 5 mass% of an acid dye, an aminoalkyl (meth)acrylate copolymer polymerized from a combination of specific monomers and having a ratio of the monomers in a predetermined range, a water-soluble organic solvent, and water, and has a pH of lower than 7.0. Applying this marker composition for skin using an applicator to a desired skin site to which the composition is desirably applied allows good color development without color separation and can also prevent absorption of the dye into the skin or the like, and can provide a beautiful finish.

### Examples

The present disclosure will be described in further detail with reference to production examples, examples, and comparative examples, but the present disclosure is not limited by the following examples and the like.

### Production Examples 1 to 7

Aminoalkyl (meth)acrylate copolymers used in Examples 1 to 3 and Comparative Examples 1 to 7 were obtained by the following production method. In the following, "part(s)" represents "part(s) by mass".

### Production Example 1

An aminoalkyl (meth) acrylate copolymer was produced by the following procedure using 18.3 parts of ethyl methacrylate, 4 parts of 2-(dimethylamino)ethyl methacrylate, 9.3 parts of 2-ethylhexyl acrylate, 1 part of azobisisobutyronitrile (V-601, available from FUJIFILM Wako Pure Chemical Corporation), and 67.4 parts of n-propyl alcohol.

A 500-milliliter flask was equipped with a stirrer, a reflux condenser, a thermometer, and a nitrogen gas inlet tube and set in a warm water bath, and the above materials were placed in the flask. The internal temperature was raised to 80°C while nitrogen gas was introduced, and solution polymerization was performed. The polymerization was terminated after 6 hours of aging, to obtain aminoalkyl (meth)acrylate copolymer [2-(dimethylamino)ethyl methacrylate/2-ethylhexyl acrylate/ethyl methacrylate copolymer].

### Production Example 2

In Production Example 1 above, 1 part of azobisisobutyronitrile (V-601, available from FUJIFILM Wako Pure Chemical Corporation) was substituted with 1 part of (AIBN, available from FUJIFILM Wako Pure Chemical Corporation), and an aminoalkyl (meth)acrylate copolymer was obtained in the same manner as in Production Example 1 above.

### Production Example 3

An aminoalkyl (meth) acrylate copolymer was obtained in the same manner as in Production Example 1 above by changing 4 parts of 2-(dimethylamino)ethyl methacrylate of Production Example 1 to 2.4 parts, and 67.4 parts of n-propyl alcohol to 69 parts (total amount 100 parts).

### Production Example 4

An aminoalkyl (meth) acrylate copolymer was obtained in the same manner as in Production Example 1 above, except for changing the amounts in Production Example 1 above to 15.8 parts of ethyl methacrylate, 7.5 parts of 2-(dimethylamino)ethyl methacrylate, and 8.3 parts of 2-ethylhexyl acrylate (total amount 100 parts).

### Production Example 5

An aminoalkyl (meth) acrylate copolymer was obtained in the same manner as in Production Example 1 above, except for changing the amounts in Production Example 1 above to 15.6 parts of ethyl methacrylate, 8 parts of 2-(dimethylamino)ethyl methacrylate, and 8 parts of 2-ethylhexyl acrylate (total amount 100 parts).

### Production Example 6

An aminoalkyl (meth) acrylate copolymer was obtained in the same manner as in Production Example 1 above, except for changing the amounts in Production Example 1 above to 10 parts of ethyl methacrylate, 15 parts of 2-(dimethylamino)ethyl methacrylate, and 5 parts of 2-ethylhexyl acrylate (total amount 100 parts).

### Production Example 7

An aminoalkyl (meth) acrylate copolymer was obtained in the same manner as in Production Example 1 above, except for changing the amounts in Production Example 1 above to 6 parts of ethyl methacrylate, 21 parts of 2-(dimethylamino)ethyl methacrylate, and 3 parts of 2-ethylhexyl acrylate (total amount 100 parts).

Marker compositions for skin were obtained by a usual method, using the blending compositions shown in Table 1 below each including aminoalkyl (meth)acrylate copolymer obtained.
The resulting marker compositions for skin were evaluated for a viscosity value, a pH value, color-developing properties, water resistance, and water-resistant adhesion by the following methods. These results are shown in Table 1 below.

### Method for measuring viscosity

Each marker composition for skin obtained by the above method was measured for viscosity under conditions of a temperature of 25°C with a cone-plate viscometer: a standard cone rotor, a cone angle of 1°34', a cone radius of 24 mm, a shear rate of 76.6 s⁻¹, TVE-25L available from Toki Sangyo Co., Ltd.

### Method for measuring pH

Each marker composition for skin obtained by the above method was measured for pH at 25°C with a glass electrode pH meter.

### Evaluation method for color-developing properties

Each resulting marker composition for skin was filled into an applicator equipped with an eyeliner/eyebrow container (UC-75, application part: nylon pen core) available from Mitsubishi Pencil Co., Ltd., applied to (a line was drawn on) the back of the hand, and evaluated for color-developing properties by the following evaluation criteria.

### Evaluation criteria:

A: Bright color development is observed.
B: Slight lightness is observed in color development.
C: Significant lightness is observed in color development.

Method for evaluating water resistance and water-resistant adhesion Each resulting marker composition for skin was filled into an applicator equipped with an eyeliner/eyebrow container (UC-75, application part: nylon pen core) available from Mitsubishi Pencil Co., Ltd., applied to (a line was drawn on) the back of the hand, and evaluated for water resistance and water-resistant adhesion by the following evaluation criteria.

Evaluation criteria for water resistance:
A: No change in the drawn line with running water.
B: Slight change is observed in the drawn line with water.
C: Significant change is observed in the drawn line with running water.

- : Cannot be evaluated.

Evaluation criteria for water-resistant adhesion:
A: No change in the drawn line due to rubbing with a ball of a finger after running water.
B: Slight change is observed in the drawn line due to rubbing with a ball of a finger after running water.
C: Significant change is observed in the drawn line due to rubbing with a ball of a finger after running water.

- : Cannot be evaluated.

**[Table 1]**

| (Total amount: 100 mass%) | | | | | | | | | | |
|---|---|---|---|---|---|---|---|---|---|---|
| | Examples | | Comparative Examples | | Examples | Comparative Examples | | | | |
| | 1 | 2 | 1 | 2 | 3 | 3 | 4 | 5 | 8 | 7 |
| Acid dye *1 | 3 | 3 | 6 | 3 | 3 | 3 | 3 | 3 | 3 | |
| Basic dye *2 | | | | | | | | | | 3 |
| Aminoalkyl (meth)acrylate copolymer [group A x group B] Production Example No. | 1 | 2 | 1 | 1 | 3 | 4 | 5 | 8 | 7 | 1 |
| Content (solid content) | 50 (15) | 50 (15) | 50 (15) | 50 (15) | 50 (15) | 50 (15) | 50 (15) | 50 (15) | 50 (15) | 50 (15) |
| Ratio of group B monomer (mass%) | 12.7 | 12.7 | 12.7 | 12.7 | 8.0 | 23.7 | 25.3 | 50.0 | 70.0 | 12.7 |
| pH adjuster (5M HCl) | 2.3 | 2.3 | 2.3 | 0 | 1.5 | 2.3 | 2.3 | 2.3 | 2.3 | 2.3 |
| N-Propyl alcohol | 30 | 30 | 30 | 30 | 30 | 30 | 30 | 30 | 30 | 30 |
| Water (purified water) | Balance | Balance | Balance | Balance | Balance | Balance | Balance | Balance | Balance | Balance |
| Viscosity (mPa·s) | 19.4 | 20.0 | 20.5 | - | 17.7 | 23.0 | 23.4 | 24.0 | 25.5 | - |
| pH | 5.4 | 5.4 | 4.8 | 8.2 | 5.8 | 6.7 | 6.8 | 7.2 | 7.5 | 5.4 |
| Color-developing properties | A | A | A | C | A | A | A | A | A | C |
| Water resistance | A | A | B | - | A | B | C | C | C | - |
| Water-resistant adhesion | A | A | A | - | A | C | C | C | C | - |

| | | | | | | | | | | |
|---|---|---|---|---|---|---|---|---|---|---|
| ***1: Blue No. 1 (C. I. 42090) + Orange No. 205 (C. I. 15510) (mass ratio 1:1)** ***2: Basic Blue 99** | | | | | | | | | | |

The results in Table 1 above revealed that Examples 1 to 3, which were included in the scope of the present disclosure, exhibited excellent color-developing properties, water resistance, and water-resistant adhesion compared with Comparative Examples 1 to 7, which were not included in the scope of the present disclosure. In addition, surprisingly, the nylon pen lead was observed after useto find that the nylon pen core was not dyed and that the drawn line density and color tone were also stable.

### Industrial Applicability

There is provided the marker composition for skin that can be easily applied to a skin site, such as a face and a body, to which the composition is desired to be applied. This marker composition for skin exhibits excellent color development without color separation and can also prevent absorption of the dye into the skin or the like, and can provide a beautiful finish. In addition, this marker composition for skin can be used also as an eyebrow cosmetic or a temporary hair dye.

## Claims

1. A marker composition for skin, the composition comprising at least 1 to 5 mass% of an acid dye, an aminoalkyl (meth) acrylate copolymer, a water-soluble organic solvent, and water, wherein a pH is lower than 7.0, the aminoalkyl (meth)acrylate copolymer is a copolymer polymerized from monomers, the monomers including a monomer selected from group A and a monomer selected from group B, and a ratio of the monomer of the group B is 2 to 20 mass% in the copolymer:
Group A:
ethyl (meth)acrylate, methyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-hexyl (meth)acrylate, 2-ethylhexyl (meth)acrylate, isooctyl (meth)acrylate, n-decyl (meth)acrylate, isododecyl (meth)acrylate, lauryl (meth)acrylate, palmityl (meth)acrylate, stearyl (meth)acrylate, behenyl (meth)acrylate, cyclohexyl (meth)acrylate, benzyl (meth)acrylate, and isobornyl (meth)acrylate; and
Group B:
2-(dimethylamino)ethyl (meth)acrylate, 2-(diethylamino)ethyl (meth)acrylate, and 3-(dimethylamino)propyl (meth)acrylate.

2. The marker composition for skin according to claim 1, wherein the acid dye is a legally-approved cosmetic dye.

3. The marker composition for skin according to claim 1 or 2, wherein the water-soluble organic solvent is a lower alcohol having 3 or less carbons.
